# EUROPEAN PATENT APPLICATION

(11) **EP 3 604 291 A1**
(43) Date of publication of application: **05.02.2020**
(21) Application number: 18788431.7
(22) Date of filing: 20.04.2018
(51) Int. Cl.: C07D 311/30, A61K 31/352, A61P 31/12

(54) **COMPOUND OR SALT THEREOF, ANTIVIRAL AGENT, AND PHARMACEUTICAL COMPOSITION**

(30) Priority: 21.04.2017 JP 2017084293
(71) Applicant: Tsuchida, Yuuzo, Tokyo 142-0062 (JP)
(72) Inventor: MURAYAMA Tsugiya, Kahoku-gun Ishikawa 920-0274 (JP); FUJIMOTO Kazuhiro, Kanazawa-shi Ishikawa 920-0944 (JP); SADANARI Hidetaka, Kanazawa-shi Ishikawa 920-1303 (JP); YAMAGUCHI Nobuo, Kahoku-gun Ishikawa 920-0267 (JP); KAWABE Mitsuo, Kawaguchi-shi Saitama 334-0056 (JP); SAKURAI Daisuke, Tokyo 142-0061 (JP); TSUCHIDA Kenjirou, Tokyo 142-0062 (JP); TSUCHIDA Yuuzo, Tokyo 142-0062 (JP)
(74) Representative: LKGLOBAL Lorenz & Kopf PartG mbB Patentanwälte
(86) International application number: PCT/JP2018/016362
(87) International publication number: WO 2018/194172

(57) **Abstract**

The present invention addresses the problem of providing a tricin derivative having higher antiviral activity. The compound according to the present invention is a compound represented by formula (I) or a salt thereof.

(In formula (I), at least one of R¹-R⁵ represents a fluorine atom, and the rest of R¹-R⁵, which are not fluorine atoms, represent a hydrogen atom or a hydroxy group.)

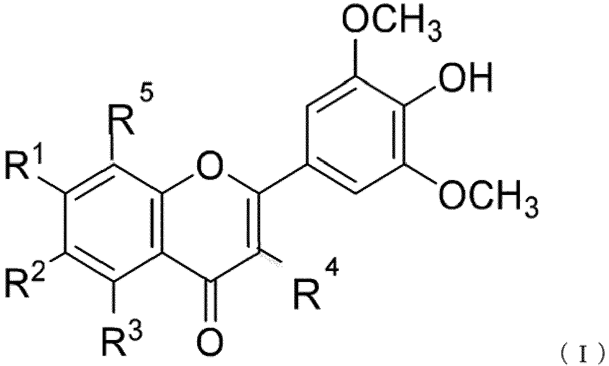

## Description

### TECHNICAL FIELD

The present invention relates to a compound or a salt thereof, an antiviral agent and a pharmaceutical composition.

### BACKGROUND ART

Tricin [4',5,7-trihydroxy-3',5'-dimethoxyflavone (tricin)], which is one of flavonoids, is known to be contained in Poaceae plants, etc. and to have various bioactivities such as antiviral effects and anticancer effects (for example, Patent Documents 1 and 2).
Patent Document 1: Japanese Unexamined Patent Application, Publication No. 2010-254649
Patent Document 2: Pamphlet of PCT International Publication No. WO2008/123102

### DISCLOSURE OF THE INVENTION

### Problems to be Solved by the Invention:

However, it had been desired that tricin can realize to have a higher antiviral activity than that of existing antiviral agents such as, for example, ganciclovir (which is known to have an antiviral activity against human cytomegalovirus).

The present invention has been made to solve the above problems, and it is an object of the present invention to provide a tricin derivative having a higher antiviral activity.

### Means for Solving the Problems:

The present inventors have found that a compound having a higher antiviral activity than that of tricin can be obtained by introducing a fluorine atom into a predetermined position of the chemical structure of tricin, thus completing the present invention. Specifically, the present invention provides the followings:
(1) A compound represented by the following formula (I) or a salt thereof: 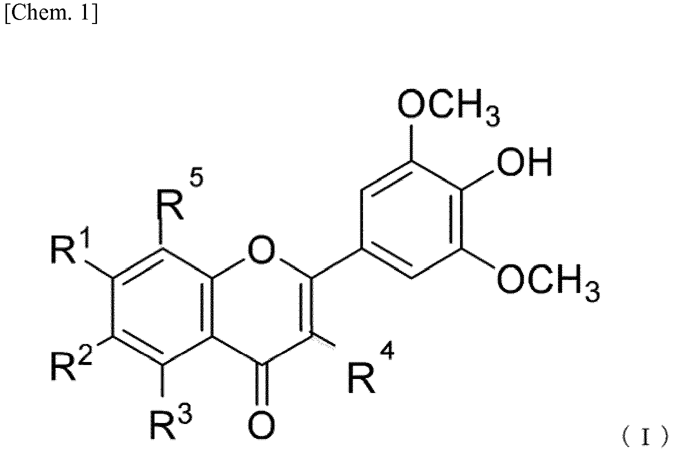 wherein, in formula (I), at least one of R¹ to R⁵ is a fluorine atom, and the rest of R¹ to R⁵, which are not fluorine atoms, are a hydrogen atom or a hydroxy group.
(2) The compound according to (1) or a salt thereof, wherein one or more of R¹ and R³ are a fluorine atom.
(3) The compound according to (1) or a salt thereof, wherein one or more of R¹, R², R⁴ and R⁵ are a fluorine atom and R³ is a hydrogen atom or a hydroxy group.
(4) The compound according to (1) or a salt thereof, wherein one or more of R², R³, R⁴ and R⁵ are a fluorine atom and R¹ is a hydrogen atom or a hydroxy group.
(5) The compound according to (1) or a salt thereof, wherein one or more of R¹, R³, R⁴ and R⁵ are a fluorine atom and R² is a hydrogen atom or a hydroxy group.
(6) The compound according to (1) or a salt thereof, wherein one or more of R¹, R², R³ and R⁵ are a fluorine atom and R⁴ is a hydrogen atom or a hydroxy group.
(7) The compound according to (1) or a salt thereof, wherein one or more of R¹, R², R³ and R⁴ are a fluorine atom and R⁵ is a hydrogen atom or a hydroxy group.
(8) An antiviral agent comprising the compound according to any one of (1) to (7) or a salt thereof.
(9) A pharmaceutical composition comprising the compound according to any one of (1) to (7) or a salt thereof.

### EFFECTS OF THE INVENTION

According to the present invention, a tricin derivative having a higher antiviral activity is provided.

### BRIEF DESCRIPTION OF THE DRAWINGS

- Fig. 1: shows an antiviral activity of the compound according to Examples.
- Fig. 2: shows an antiviral activity of the compound according to Examples.
- Fig. 3: shows evaluation results of cytotoxicity of the compound according to Examples.

### PREFERRED MODE FOR CARRYING OUT THE INVENTION

Embodiments of the present invention will be described below, but the present invention is not particularly limited thereto.

### <Compound>

A compound of the present invention is represented by the following formula (I): 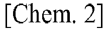 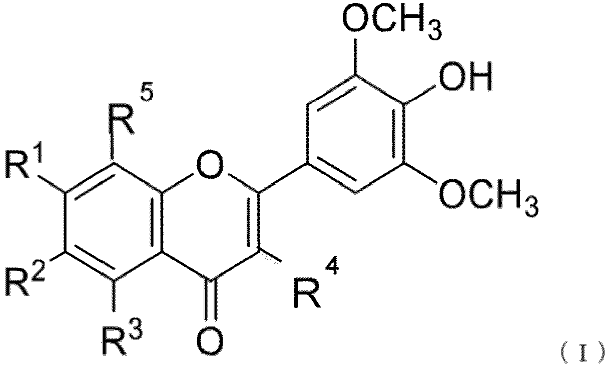 wherein, in formula (I), at least one of R¹ to R⁵ is a fluorine atom, and the rest of R¹ to R⁵, which are not fluorine atoms, are a hydrogen atom or a hydroxy group.

In the above formula (I), a compound in which one or more of R¹ and R³ are a fluorine atom is preferable in terms of the fact that a compound having a high antiviral activity is easily obtained. In the above formula (I), the following compounds are preferable in terms of the fact that a compound having a particularly high antiviral activity is easily obtained. A compound in which one or more of R¹, R², R⁴ and R⁵ are a fluorine atom and R³ is a hydrogen atom or a hydroxy group. A compound in which one or more of R², R³, R⁴ and R⁵ are a fluorine atom and R¹ is a hydrogen atom or a hydroxy group. A compound in which one or more of R¹, R³, R⁴ and R⁵ are a fluorine atom and R² is a hydrogen atom or a hydroxy group. A compound in which one or more of R¹, R², R³ and R⁵ are a fluorine atom and R⁴ is a hydrogen atom or a hydroxy group. A compound in which one or more of R¹, R², R³ and R⁴ are a fluorine atom and R⁵ is a hydrogen atom or a hydroxy group.

Among the compounds represented by formula (I), a compound in which R¹ is a fluorine atom and R² and R³ are a hydrogen atom and a compound in which R² is a fluorine atom and R¹ and R³ are a hydrogen atom are preferable in terms of the fact that a compound having a remarkably high antiviral activity than that of tricin is easily obtained.

The compound represented by the above formula (I) may be in the form of a salt. The salt as the compound represented by the above formula (I) is not particularly limited, and it is preferably a pharmaceutically acceptable salt. Examples thereof include salts of inorganic acids (e.g., hydrochloric acid, hydrobromic acid, hydroiodic acid, sulfuric acid, nitric acid, and phosphoric acid), salts of organic acids (e.g., formic acid, acetic acid, propionic acid, citric acid, tartaric acid, fumaric acid, butyric acid, oxalic acid, succinic acid, malonic acid, maleic acid, lactic acid, malic acid, carbonic acid, glutamic acid, aspartic acid, methanesulfonic acid, benzenesulfonic acid, and p-toluenesulfonic acid), salts of alkali metals (e.g., sodium and potassium), salts of alkaline earth metals (e.g., calcium and magnesium), salts of metals (e.g., iron and zinc) and the like. These pharmaceutically acceptable salts can be prepared according to conventional methods.

Tricin is known to have an antiviral effect depending on host factors (particularly, chemokines). Its mechanism of action is that the antiviral effect is exerted by inhibiting viral DNA synthesis, which is entirely different from the mechanism of action of existing antiviral agents (e.g., ganciclovir). Since the compound of the present invention has a similar mechanism of action to that of tricin while having a higher antiviral activity than that of tricin, it is expected to be used as a novel antiviral agent having a high viral activity while having a mechanism of action different from that of ganciclovir or the like.

The antiviral activity of the compound of the present invention can be evaluated by the method of Examples.

### <Method for Producing Compound>

One example of a method for producing the compound of the present invention will be described below. However, the method for producing the compound of the present invention is not limited thereto, and can be appropriately changed according to the purpose.

An example of a method for producing a compound in which R¹ is a fluorine atom and R², R³, R⁴ and R⁵ are a hydrogen atom among compounds represented by formula (I) will be shown below. Compounds other than the following compound can be produced by appropriately changing the following scheme. For example, modification to produce a target compound among compounds represented by formula (I) may be performed between or before and after each step, and modification to produce a target compound may be performed after production of the following compound.

The meanings of the abbreviations in the following formula are as follows:
Me: Methyl group
TBS: tert-Butyldimethylsilyl group
LiHMDS: Lithium bis(trimethylsilyl)amide
THF: Tetrahydrofuran
AcOH: Acetic acid

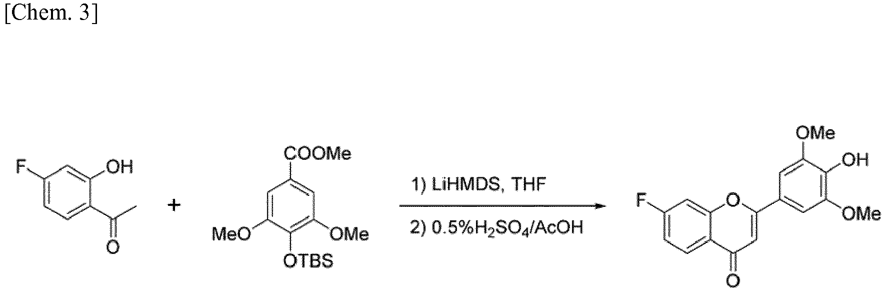

### <Antiviral Agent>

Since the compound of the present invention has a high antiviral activity, it can be suitably used as a component of an antiviral agent. "Virus" in the present invention includes animal virus, insect virus, plant virus, bacterial virus (bacteriophages), and particularly, means a virus that has unfavorable effects on plants and animals. "Having an antiviral activity" in the present invention means having a killing effect on the above virus or having a growth inhibitory effect on the above virus.

The antiviral agent of the present invention is also effective as an antiviral agent for humans, mammals other than humans, birds, fishes and shellfishes, crustaceans, insects, amphibians, reptiles and the like. Therefore, the antiviral agent of the present invention can be used as an antiviral agent for these animals (e.g., pharmaceuticals for pets, feeds for animals, pet foods and the like). Furthermore, the antiviral agent of the present invention is also useful as an antiviral agent for various plants in addition to animals, and is also useful as an antiseptic.

Examples of a virus for which the antiviral agent of the present invention is particularly effective include cytomegalovirus of DNA virus (e.g., human cytomegalovirus, monkey cytomegalovirus and mouse cytomegalovirus), herpes simplex virus (e.g., human herpes simplex virus type 1, human herpes simplex virus type 2 and fish herpes simplex virus), varicella-zoster virus, hepatitis B virus, and influenza virus of RNA virus (influenza virus A, influenza virus B and influenza virus C), Zika virus and the like.

The antiviral agent of the present invention may contain a compound represented by the above formula (I) and/or a salt thereof. When a salt of a compound represented by the above formula (I) is contained, one salt may be used alone, or two or more salts may be used in combination. The antiviral agent of the present invention may be formulated. The dosage form is not particularly limited, and examples thereof include tablets, pills, powders, solutions, suspensions, emulsions, granules, capsules, ointments and the like. As a carrier used for formulation, diluents and excipients which are commonly used for usual drugs can be used, and examples thereof include fillers, expanders, binders, disintegrants, surfactants, lubricants and the like. The antiviral agent of the present invention may be any of an orally administered agent, a parenterally administered agent and a locally administered agent. Examples of the orally administered agent include tablets, pills, dusts, powders, suspensions, emulsions, granules, capsules, solutions, various drinks, various foods (e.g., chewing gums, candies, chocolates, breads, cookies, rice crackers, biscuits, buckwheat noodles, udon noodles, jellies, miso soups, soups and potages) and the like. Examples of the parenterally administered agent include injections, suppositories and the like. Examples of the locally administered agent include creams, ointments, film agents, carriers (e.g., gauze made of natural fibers or synthetic fibers) impregnated with the compound of the present invention, cosmetics (e.g., lipsticks) containing the compound of the present invention and the like.

The antiviral agent of the present invention may contain publicly known components (e.g., coloring agents, preservatives, fragrances, flavoring agents, sweetening agents, base components, oil components, moisturizers, antiseptics, stabilizers and surfactants) as needed. The antiviral agent of the present invention may be used in combination with other antiviral agents.

The amount of the compound of the present invention contained in the antiviral agent is not particularly limited, and can be appropriately selected according to the effect to be obtained. For example, preferably 0.5 to 50% by mass and more preferably 1.0 to 8.0% by mass of the compound of the present invention may be contained in the antiviral agent. Also, preferably 1 ppm to 1,000 ppm (solid content concentration) of the compound of the present invention may be contained in the antiviral agent. When the antiviral agent of the present invention is used for humans, the antiviral agent may be prepared so that preferably 0.01 to 500 mg, more preferably 0.5 to 200 mg and still more preferably 1 to 100 mg based on a body weight of 1 kg per day of the compound of the present invention can be administered. With respect to the dosage form, administration may be performed once or about two to six times in divided doses by considering the symptoms, age, body weight and the like of a subject to be administered.

The method for administering the antiviral agent of the present invention can be selected according to the form of a preparation, age and sex of a subject to be administered, the degree of the symptoms of the subject to be administered and other conditions.

The method for producing the antiviral agent of the present invention is not particularly limited, and a method for appropriately mixing and stirring the above component and a publicly known formulation method can be adopted.

### Pharmaceutical Composition>

A pharmaceutical composition of the present invention includes the compound of the present invention. The constitution, the administration method, the production method and the like of the pharmaceutical composition of the present invention are the same as those of the above antiviral agent.

The pharmaceutical composition of the present invention can be used as a composition for mucosal protection, a composition for prevention and/or treatment of viral infection and the like. According to the pharmaceutical composition of the present invention, a protective effect on the mucosa such as, for example, eye, nose, throat, ear, anus and genital part and an inhibitory effect on invasion of virus from a wound site and skin into the body are exerted.

### EXAMPLES

The present invention will be more specifically described by way of Examples below, but the present invention is not limited to these Examples.

### <Synthesis Example>

A compound represented by the following formula (7-fluoro-4'-hydroxy-3',5'-dimethoxyflavone) was synthesized by the following method. The compound is a compound in which R¹ is a fluorine atom and R², R³, R⁴ and R⁵ are a hydrogen atom in the above formula (I).

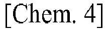

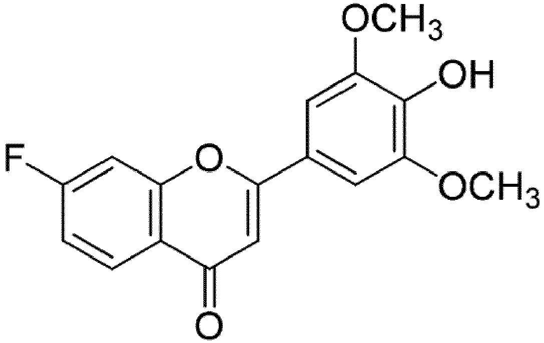

In tetrahydrofuran (10 ml), 200 mg (0.61 mmol) of 4-O-tert-butyldimethylsilyl-3,5-dimethoxybenzoate and 188 mg (1.22 mmol) of 4'-fluoro-2'-hydroxyacetophenone were dissolved. To the solution thus obtained, 4.88 ml of a tetrahydrofuran solution of 1M lithium bis(trimethylsilyl)amide was added, followed by stirring at room temperature for one day. Then, 10% aqueous hydrochloric acid solution was added to the mixture thus obtained, and the mixture was extracted with ethyl acetate twice and washed with saturated saline, followed by drying over anhydrous sodium sulfate. After evaporating the solvent, the residue thus obtained was sufficiently dried using a vacuum pump overnight. Next, the solid thus obtained was dissolved in 0.5% sulfuric acid-containing acetic acid, and reacted at 100°C for 3 hours. Distilled water was poured into the reactant thus obtained, and the reactant was extracted with ethyl acetate twice and washed with saturated saline, followed by drying over anhydrous sodium sulfate. After evaporating the solvent, the residue thus obtained was purified by silica gel column chromatography (chloroform: methanol = 100:1) to obtain 137 mg (yield of 71%) of 7-fluoro-4'-hydroxy-3',5'-dimethoxyflavone. The results of NMR spectrum analysis are shown below.
¹H NMR(400MHz, DMSO-d₆): δ9.33(s, 1H, OH), 8.08(dd, 1H, J=9.2 and 6.4Hz, H-5), 7.80(dd, 1H, J=10.1 and 2.8Hz, H-8), 7.40-7.32(m, 3H, H-2', H-6 and H-6'), 7.09(s, 1H, H-3),3.90(s, 6H, 2OMe).

### <Evaluation of Antiviral Activity - 1>

Using the compound obtained as mentioned above (the compound of the present invention) and tricin, the antiviral activity of each compound was evaluated in accordance with the following procedure.

Diploid fibroblasts derived from human embryonic lung (Human embryonic lung fibroblasts; HEL cells) cultured in a 9.6 cm² petri dish until they became confluent using Dulbecco's Modified Eagle Medium (DMEM) to which 8% fetal calf serum (FCS) was added were prepared. The HEL cells were adsorbed and infected with a Towne strain of human cytomegalovirus (HCMV) with a multiplicity of infection (MOI) of 1, and cultured at 37°C for 1 hour to obtain HCMV-infected cells.

The compound of the present invention or tricin which were serially diluted with dimethyl sulfoxide (compound concentration: 0.001, 0.01, 0.1, 1 and 10 µM) was added to the above HCMV-infected cells and cultured for 6 days. The number of infectious HCMV particles produced in the culture supernatant (viral load) was determined by the plaque method.

The plaque method was performed in the following manner. After the HEL cells cultured in a 24-well plate until they became confluent were adsorbed and infected with the culture supernatant which was 10-fold serially diluted at 37°C for 1 hour, the 2% FCS and 0.4% agar-containing DMEM media were overlaid and then cultured. Several days (6 to 12 days) after the initiation of culture, the number of plaques appeared was counted under a microscope, and the viral load was measured.

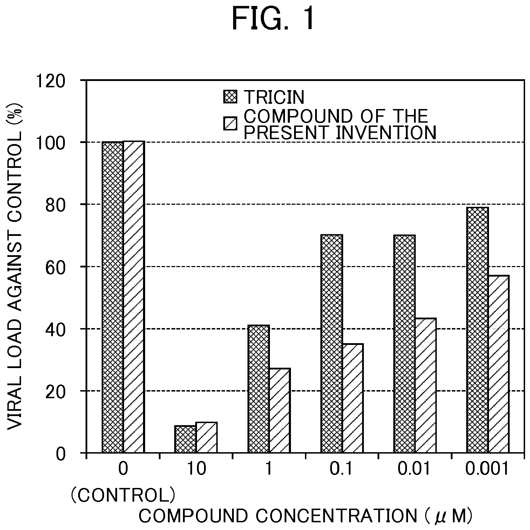

Fig. 1 shows the evaluation results of the antiviral activity possessed by each compound based on the viral load determined by the plaque method. The antiviral activity possessed by each compound was represented as a relative value of the viral load in the case where each compound was added to the HCMV-infected cells when the viral load in the "control" in which only dimethyl sulfoxide was added to the HCMV-infected cells (i.e., the viral load in the case where "compound concentration" in Fig. 1 is 0) was regarded as 100. A lower value of the relative value (the viral load against control) means a higher antiviral activity.

As shown in Fig. 1, the compound of the present invention showed a remarkably higher antiviral activity than tricin. An advantageous effect of the compound of the present invention on tricin was clearly observed, particularly, at compound concentrations in the range of 0.001 to 1.000 µM.

### <Evaluation of Antiviral Activity - 2>

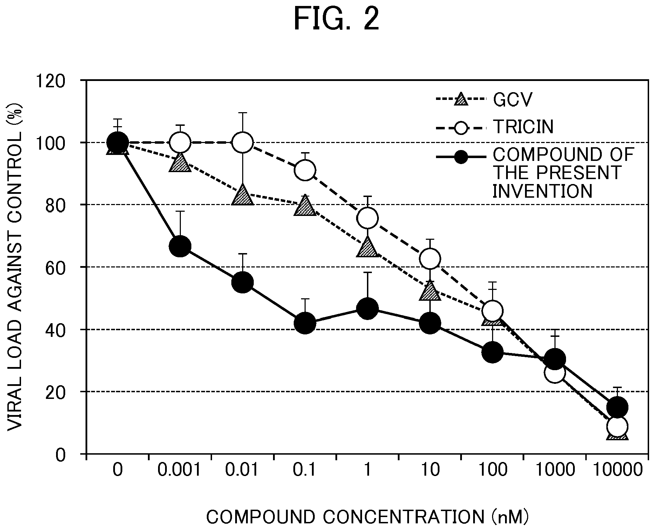

Using the compound obtained as mentioned above (the compound of the present invention), tricin and ganciclovir, the antiviral activity of each compound was evaluated in the same manner as in the above <Evaluation of Antiviral Activity - 1>. The results are shown in Fig. 2.

As shown in Fig. 2, the compound of the present invention showed a remarkably higher antiviral activity than tricin and ganciclovir. An advantageous effect of the compound of the present invention was clearly observed, particularly, at compound concentrations in the range of 0.1 to 1,000 nM.

The ECso value of the compound of the present invention, tricin and ganciclovir was 0.13 nM, 54.3 nM and 27.5 nM, respectively. In this way, the compound of the present invention showed an anti-HCMV effect which was about 400-fold higher than that of tricin and about 200-fold higher than that of ganciclovir.

### <Evaluation of Cytotoxicity>

Using the compound obtained as mentioned above (the compound of the present invention), tricin and flavopiridol, the cytotoxicity to the HEL cells was evaluated in accordance with the following procedure. Flavopiridol is a control compound in this Test Example, which is known to have toxicity although it functions as an antiviral agent.

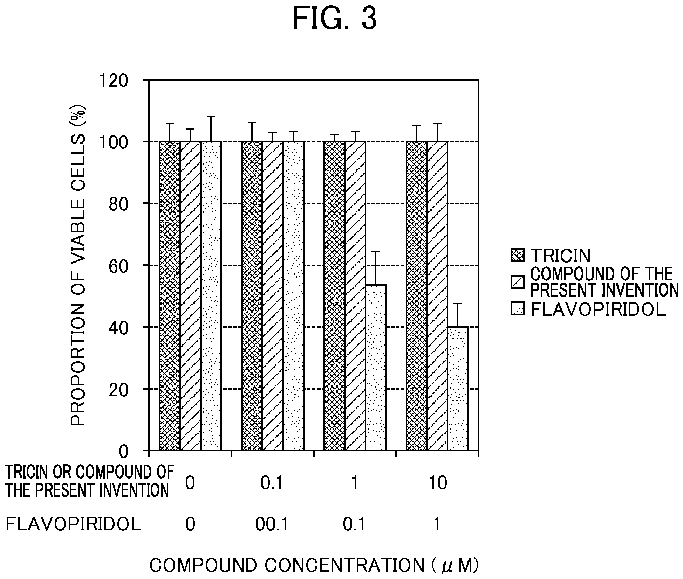

The HEL cells were cultured at 37°C for 5 days in the presence or absence of the above each compound. The proportion of viable cells after culture was calculated by the LDH release assay. The kit (trade name "Cyto Tox 96 (registered trademark) assay", manufactured by Promega Corporation) was used for the assay. The test was repeated three times, and data were represented as mean ± SD. The results are shown in Fig. 3.

As shown in Fig. 3, no cytotoxicity by the compound of the present invention was observed at concentrations of at least up to 10 µM.

## Claims

1. A compound represented by the following formula (I) or a salt thereof: 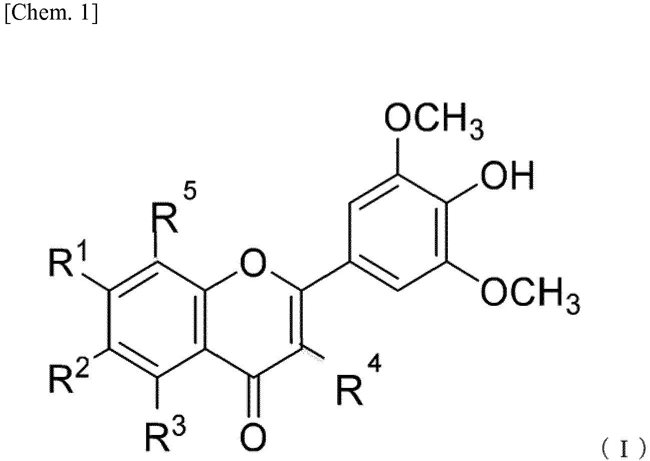 wherein, in formula (I), at least one of R¹ to R⁵ is a fluorine atom, and the rest of R¹ to R⁵, which are not fluorine atoms, are a hydrogen atom or a hydroxy group.

2. The compound according to claim 1 or a salt thereof, wherein one or more of R¹ and R³ are a fluorine atom.

3. The compound according to claim 1 or a salt thereof, wherein one or more of R¹, R², R⁴ and R⁵ are a fluorine atom and R³ is a hydrogen atom or a hydroxy group.

4. The compound according to claim 1 or a salt thereof, wherein one or more of R², R³, R⁴ and R⁵ are a fluorine atom and R¹ is a hydrogen atom or a hydroxy group.

5. The compound according to claim 1 or a salt thereof, wherein one or more of R¹, R³, R⁴ and R⁵ are a fluorine atom and R² is a hydrogen atom or a hydroxy group.

6. The compound according to claim 1 or a salt thereof, wherein one or more of R¹, R², R³ and R⁵ are a fluorine atom and R⁴ is a hydrogen atom or a hydroxy group.

7. The compound according to claim 1 or a salt thereof, wherein one or more of R¹, R², R³ and R⁴ are a fluorine atom and R⁵ is a hydrogen atom or a hydroxy group.

8. An antiviral agent comprising the compound according to any one of claims 1 to 7 or a salt thereof.

9. A pharmaceutical composition comprising the compound according to any one of claims 1 to 7 or a salt thereof.
